# EUROPÄISCHE PATENTSCHRIFT

(11) **EP 3 231 380 B1**
(45) Veröffentlichungstag und Bekanntmachung des Hinweises auf die Patenterteilung: **04.05.2022**
(21) Anmeldenummer: 17163573.3
(22) Anmeldetag: 29.03.2017
(51) Int. Cl.: A61B 17/29

(54) **WERKZEUG FÜR EIN MEDIZINISCHES INSTRUMENT UND MEDIZINISCHES INSTRUMENT**
TOOL FOR A MEDICAL INSTRUMENT AND MEDICAL INSTRUMENT
OUTIL POUR UN INSTRUMENT MÉDICAL ET INSTRUMENT MÉDICAL

(30) Priorität: 14.04.2016 DE 102016106930
(43) Veröffentlichungstag der Anmeldung: 18.10.2017
(73) Patentinhaber: Karl Storz SE & Co. KG, 78532 Tuttlingen (DE)
(72) Erfinder: Besse, Régis, 78610 Le Perray en Yvelines (FR); Thouément, Yann, 78690 Les Essarts-Le-Roi (FR)

(56) Entgegenhaltungen:
- WO-A1-2010/144219
- WO-A2-2012/051200
- WO-A2-2012/083041

## Beschreibung

Die vorliegende Erfindung ist auf ein Werkzeug für ein medizinisches Instrument und auf ein medizinisches Instrument mit einem solchen Werkzeug bezogen. Das Werkzeug und das medizinische Instrument sind insbesondere für mikroinvasive Anwendungen vorgesehen und ausgebildet.

Insbesondere bei mikroinvasiven Anwendungen werden immer kleinere medizinische Instrumente verwendet. Um die medizinischen Instrumente durch immer kleinere Zugänge oder durch dünne Arbeitskanäle in anderen, größeren medizinischen Instrumenten einführen zu können, müssen ihre Schäfte und ihre Werkzeuge an den distalen Enden der Schäfte immer kleinere Querschnitte aufweisen. Gleichzeitig soll ein hochwertiges medizinisches Instrument auf einfache Weise vollständig gereinigt und möglichst oft wieder verwendet werden können. Die erforderliche mechanische Robustheit setzt eine einfache, möglichst wenig komplexe Gestaltung des medizinischen Instruments aus möglichst wenigen Bauteilen voraus.

WO 2012/051200 A2 zeigt ein medizintechnisches Werkzeug einem feststehenden Bauteil und einer Branche, die relativ zu diesem Bauteil um eine Achse schwenkbar ist, sowie mit einer Einrichtung zum Übertragen einer Kraft zu der schwenkbaren Branche, die über eine Kopplungseinrichtung mit der Übertragungseinrichtung gekoppelt ist.

Dieses Dokument gewährleistet somit, dass eine translatorisch aufgebrachte Kraft über Translation der Übertragungseinrichtung in eine Schwenkbewegung der schwenkbaren Branche übertragen wird. Dabei wird diese Kraft-Drehmoment-Übertragung mittels einer Quasi-Zahnstange auf ein Quasi-Zahnrad.

Eine Aufgabe der vorliegenden Erfindung besteht darin, ein verbessertes Werkzeug für ein medizinisches Instrument und ein verbessertes medizinisches Instrument zu schaffen.

Diese Aufgabe wird durch die Gegenstände der unabhängigen Ansprüche gelöst.

Weiterbildungen sind in den abhängigen Ansprüchen angegeben.

Ein Werkzeug für ein medizinisches Instrument umfasst ein feststehendes Bauteil, eine Branche, die relativ zu dem feststehenden Bauteil um eine Schwenkachse schwenkbar ist, eine Übertragungseinrichtung zum Übertragen einer Kraft zu der schwenkbaren Branche und eine Kopplungseinrichtung zum Koppeln der Übertragungseinrichtung mit der Branche derart, dass eine Translation der Übertragungseinrichtung mit einer Schwenkbewegung der schwenkbaren Branche um ihre Schwenkachse einhergeht, wobei die Kopplungseinrichtung mehrere Kopplungsabschnitte an der schwenkbaren Branche und mehrere Kopplungsabschnitte an der Übertragungseinrichtung umfasst, wobei jeder Kopplungsabschnitt an der schwenkbaren Branche einem korrespondierenden Kopplungsabschnitt an der Übertragungseinrichtung zugeordnet ist, und wobei die Kopplungsabschnitte so angeordnet und ausgebildet sind, dass die Kopplung von schwenkbarer Branche und Übertragungseinrichtung abhängig von den Positionen von schwenkbarer Branche und Übertragungseinrichtung durch unterschiedliche Paare korrespondierender Kopplungsabschnitte erfolgt.

Das Werkzeug ist insbesondere für ein mikroinvasives medizinisches Instrument vorgesehen und ausgebildet. Beispielsweise ist das Werkzeug zur zerstörungsfrei lösbaren Kopplung oder zur dauerhaften, nicht zerstörungsfrei lösbaren Verbindung mit einem distalen Ende eines Schafts eines mikroinvasiven medizinischen Instruments vorgesehen und ausgebildet.

Das Werkzeug umfasst insbesondere zwei oder mehr Branchen oder Maulteile, die zum Halten, Greifen, Quetschen oder Schneiden von Gewebe ausgebildet sein können. Dazu sind insbesondere alle oder mehrere Branchen des Werkzeugs relativ zueinander um eine oder mehrere Schwenkachsen schwenkbar. Wenn eine Branche des Werkzeugs feststehend bzw. nicht bewegbar bzw. starr ausgebildet ist, ist diese Branche insbesondere Teil des feststehenden Bauteils oder mit diesem einteilig oder monolithisch ausgebildet.

Die Schwenkachse, um die die schwenkbare Branche schwenkbar ist, ist insbesondere durch ein Gelenk zwischen der schwenkbaren Branche und dem feststehenden Bauteil definiert. Die Schwenkachse ist insbesondere orthogonal zu einer vorgesehenen Bewegungsrichtung der Übertragungseinrichtung und/oder orthogonal zu einer Längsachse des Werkzeugs und/oder orthogonal zu der Längsachse eines mit dem Werkzeug verbundenen Schafts oder eines mit dem Werkzeug lösbar verbindbaren und in der vorgesehenen Weise verbundenen Schafts. Im Fall eines gekrümmten Schafts ist die Längsachse des Schafts nahe dessen distalem Ende gemeint.

Das feststehende Bauteil umfasst insbesondere eine Schaftkupplung zur zerstörungsfrei lösbaren mechanischen Verbindung des Werkzeugs mit dem distalen Ende eines Schafts eines medizinischen Instruments oder mit dem distalen Ende eines Schafts für ein medizinisches Instrument.

Die Übertragungseinrichtung kann zur Übertragung einer Kraft und zur Übertragung eines Drehmoments vorgesehen sein. Die Übertragungseinrichtung umfasst insbesondere eine starre oder biegeflexible Stange oder ein starres oder biegeflexibles Rohr. Die Übertragungseinrichtung ist insbesondere in einem mit dem Werkzeug verbundenen Schaft angeordnet oder zur Anordnung in einem mit dem Werkzeug zu verbindenden Schaft vorgesehen und ausgebildet.

Die Kopplungsabschnitte an der Übertragungsrichtung sind insbesondere an oder nahe dem distalen Ende der Übertragungseinrichtung angeordnet. Die Kopplungsabschnitte an der schwenkbaren Branche sind insbesondere an einem Bereich der schwenkbaren Branche vorgesehen, der von dem zur Wechselwirkung mit Gewebe vorgesehenen Ende der schwenkbaren Branche abgewandt ist. Insbesondere sind mindestens zwei oder mindestens drei Paare korrespondierender Kopplungsabschnitte vorgesehen, die in mindestens zwei oder mindestens drei verschiedenen Bereichen von Positionen der schwenkbaren Branche und der Übertragungseinrichtung die schwenkbare Branche mit der Übertragungseinrichtung koppeln.

Die Kopplungsabschnitte an der Übertragungseinrichtung sind insbesondere zumindest teilweise konkav. Die Kopplungsabschnitte an der schwenkbaren Branche sind insbesondere zumindest teilweise konvex. Alternativ können die Kopplungsabschnitte an der Übertragungseinrichtung zumindest teilweise konvex und die Kopplungsabschnitte an der schwenkbaren Branche zumindest teilweise konkav sein. Alternativ können ein Teil der Kopplungsabschnitte an der Übertragungseinrichtung konkav, ein anderer Teil der Kopplungsabschnitte an der Übertragungseinrichtung konvex, ein Teil der Kopplungsabschnitte an der schwenkbaren Branche konvex und ein anderer Teil der Kopplungsabschnitte an der schwenkbaren Branche konkav sein.

Die Kopplung zwischen der Übertragungseinrichtung und der Branche entsteht insbesondere dadurch, dass jederzeit mindestens ein Kopplungsabschnitt an der schwenkbaren Branche in einen korrespondierenden Kopplungsabschnitt an der Übertragungseinrichtung und/oder ein Kopplungsabschnitt an der Übertragungseinrichtung in einen korrespondierenden Kopplungsabschnitt an der schwenkbaren Branche eingreift. Die Kopplung zwischen der schwenkbaren Branche und der Übertragungseinrichtung erfolgt insofern formschlüssig.

Bei einem Werkzeug, wie es hier beschrieben ist, umfasst die Kopplungseinrichtung insbesondere kein Pleuel.

Die Kopplungseinrichtung des hier beschriebenen Werkzeugs ermöglicht eine Kopplung der Übertragungseinrichtung mit der schwenkbaren Branche ohne einen Pleuel. Dies kann die Reduzierung der Anzahl der Bauteile des Werkzeugs ermöglichen. Die reduzierte Anzahl von Bauteilen kann bei gleichem äußerem Querschnitt des Werkzeugs größere Querschnitte der einzelnen Bauteile ermöglichen. Indem bei verschiedenen Positionen der schwenkbaren Branche - und entsprechend verschiedenen Positionen der Übertragungseinrichtung - die Kopplung durch unterschiedliche Paare korrespondierender Kopplungsabschnitte erfolgt, kann ferner das Übersetzungsverhältnis bzw. das Verhältnis zwischen Drehmoment an der schwenkbaren Branche und Kraft an der Übertragungseinrichtung eingestellt werden. Insbesondere kann auch die Abhängigkeit dieses Übersetzungsverhältnisses von der Position der schwenkbaren Branche eingestellt werden. Insbesondere kann auch bei einem großen maximalen Öffnungswinkel zwischen den Branchen des Werkzeugs für jede Position der schwenkbaren Branche ein vorteilhaftes Übersetzungsverhältnis eingestellt werden. Dazu werden insbesondere die Abstände der Kopplungsabschnitte - genauer: die Abstände der Punkte oder Linien oder Flächen, in denen korrespondierende Kopplungsabschnitte einander berühren - von der Schwenkachse eingestellt. Ein großer Abstand ermöglicht ein großes Drehmoment an der Branche, ein kleiner Abstand ermöglicht eine Schwenkbewegung der schwenkbaren Branche um einen vergleichsweise großen Winkel bei einer vergleichsweise kleinen Bewegung der Übertragungseinrichtung.

Bei einem Werkzeug, wie es hier beschrieben ist, bilden die Kopplungsabschnitte an der Übertragungseinrichtung insbesondere eine regelmäßige oder unregelmäßige Zahnstange, in die die Kopplungsabschnitte an der schwenkbaren Branche wie Zähne eines Zahnrads eingreifen.

Bei einem Werkzeug, wie es hier beschrieben ist, schließen die extremen Winkelpositionen der schwenkbaren Branche insbesondere einen Winkel von mindestens 90 Grad oder von mindestens 100 Grad oder von mindestens 110 Grad ein.

Bei vielen herkömmlichen Werkzeugen für medizinische Instrumente oder an medizinischen Instrumenten schließen die extremen Winkelpositionen der einzelnen schwenkbaren Branche einen Winkel von nur ca. 60 Grad oder ca. 70 Grad ein. Ein größerer Winkel zwischen extremen Winkelpositionen der schwenkbaren Branche und ein entsprechend größerer Öffnungswinkel können für viele Anwendungen vorteilhaft sein.

Bei einem Werkzeug, wie es hier beschrieben ist, weisen zwei benachbarte Kopplungsabschnitte an der schwenkbaren Branche bezogen auf die Schwenkachse insbesondere einen Winkelabstand im Bereich von 50 Grad bis 70 Grad oder im Bereich von 55 Grad bis 65 Grad auf.

Bei einem Werkzeug, wie es hier beschrieben ist, weist der Winkelbereich, innerhalb dessen die Kopplung zwischen der Übertragungseinrichtung und der schwenkbaren Branche durch ein ausgewähltes Paar korrespondierender Kopplungsabschnitte erfolgt, insbesondere eine Breite im Bereich von 50 Grad bis 70 Grad oder im Bereich von 55 Grad bis 65 Grad auf.

Ein Winkelabstand oder eine Breite des Winkelbereichs von ca. 60 Grad kann es ermöglichen, dass innerhalb eines Schwenkbereichs der schwenkbaren Branche von 120 Grad der Winkel zwischen der Längsachse der Übertragungseinrichtung, entlang derer diese bewegbar ist, einerseits und einer Geraden durch die Schwenkachse und dem momentan die Kopplung zwischen schwenkbarer Branche und Übertragungseinrichtung bewirkenden Kopplungsabschnitt an der schwenkbaren Branche andererseits jederzeit im Bereich zwischen 60 Grad und 120 Grad liegt. Der das Übersetzungsverhältnis bestimmende Abstand einer Geraden parallel zur Bewegungsrichtung der Übertragungseinrichtung durch den die Kopplung bewirkenden Kopplungsabschnitt an der schwenkbaren Branche einerseits und der Schwenkachse der schwenkbaren Branche andererseits variiert dann um nicht mehr als das Verhältnis 1 : sin(60 Grad) = 1 : 0,87.

Bei einem Werkzeug, wie es hier beschrieben ist, umfasst ein Kopplungsabschnitt an der Übertragungseinrichtung insbesondere eine Nut oder einen Schlitz in der Übertragungseinrichtung.

Insbesondere umfasst jeder Kopplungsabschnitt an der Übertragungseinrichtung eine Nut oder einen Schlitz in der Übertragungseinrichtung. Die Nut oder der Schlitz bzw. die Nuten oder Schlitze sind insbesondere an oder nahe dem distalen Ende der Übertragungseinrichtung angeordnet.

Jede Nut oder jeder Schlitz erstreckt sich insbesondere in einer Richtung orthogonal oder im Wesentlichen orthogonal zur vorgesehenen Bewegungsrichtung der Übertragungseinrichtung. Jede Nut oder jeder Schlitz an der Übertragungseinrichtung kann gerade oder im Wesentlichen gerade oder gekrümmt sein. Insbesondere ist eine Nut oder ein Schlitz gerade und eine Nut oder ein Schlitz gekrümmt. Beispielsweise ist eine erste Nut gerade, und eine zweite, distal der ersten Nut angeordnete zweite Nut ist zu der ersten Nut hin gekrümmt; oder ein erster Schlitz ist gerade, und ein zweiter, distal des ersten Schlitzes angeordneter zweiter Schlitz ist zu dem ersten Schlitz hin gekrümmt.

Bei einem Werkzeug, wie es hier beschrieben ist, umfasst ein Kopplungsabschnitt an der schwenkbaren Branche insbesondere einen Zapfen oder einen Stift oder einen stabförmigen Abschnitt.

Insbesondere umfasst jeder Kopplungsabschnitt an der schwenkbaren Branche einen Zapfen oder einen Stift oder einen stabförmigen Abschnitt.

Bei einem Werkzeug, wie es hier beschrieben ist, umfasst insbesondere ein Kopplungsabschnitt oder jeder Kopplungsabschnitt an der schwenkbaren Branche eine Nut oder einen Schlitz in der schwenkbaren Branche.

Bei einem Werkzeug, wie es hier beschrieben ist, umfasst insbesondere ein Kopplungsabschnitt oder jeder Kopplungsabschnitt an der Übertragungseinrichtung einen Zapfen oder einen Stift oder einen stabförmigen Abschnitt an der Übertragungseinrichtung.

Bei einem Werkzeug, wie es hier beschrieben ist, sind insbesondere jeweils zwei als Zapfen ausgebildete Kopplungsabschnitte an der schwenkbaren Branche einander gegenüber angeordnet, wobei jeweils zwei als Nuten ausgebildete Kopplungsabschnitte an der Übertragungseinrichtung einander gegenüber angeordnet sind.

Die als Zapfen ausgebildeten Kopplungsabschnitte sind insbesondere einander zugewandt an zwei einander zugewandten Oberflächen zweier wandförmiger Bereiche der schwenkbaren Branche angeordnet, wobei die Übertragungseinrichtung zwischen den wandförmigen Bereichen der schwenkbaren Branche angeordnet ist. Insbesondere sind jeweils zwei als Zapfen ausgebildete Kopplungsabschnitte spiegelsymmetrisch zu einer Symmetrieebene angeordnet und ausgebildet, wobei die Symmetrieebene gleichzeitig Symmetrieebene der Übertragungseinrichtung ist.

Als Nuten ausgebildete Kopplungsabschnitte an der Übertragungseinrichtung sind insbesondere an voneinander abgewandten Seiten und in voneinander abgewandten Oberflächen der Übertragungseinrichtung ausgebildet. Jeweils zwei als Nuten ausgebildete Kopplungsabschnitte sind insbesondere spiegelsymmetrisch zu einer Symmetrieebene angeordnet, wobei die Symmetrieebene insbesondere gleichzeitig Symmetrieebene der schwenkbaren Branche ist.

Bei einem Werkzeug, wie es hier beschrieben ist, sind die Kopplungsabschnitte insbesondere paarweise spiegelsymmetrisch zu einer Symmetrieebene angeordnet.

Insbesondere sind alle Kopplungsabschnitte paarweise spiegelsymmetrisch zu einer Symmetrieebene angeordnet.

Eine symmetrische Anordnung von Kopplungsabschnitten kann eine Führung der Übertragungseinrichtung bzw. von dessen distalem Ende in einem korrespondierenden Spalt in der Branche oder umgekehrt eine Führung der Branche in einem Spalt im distalen Ende der Übertragungseinrichtung ermöglichen. Diese Führung kann das Spiel verringern und die mechanische Robustheit des Werkzeugs verbessern.

Bei einem Werkzeug, wie es hier beschrieben ist, ist insbesondere eine Wand zwischen einander gegenüberliegenden Nuten an der Übertragungseinrichtung angeordnet.

Selbst eine dünne Wand zwischen einander gegenüberliegenden und voneinander abgewandten Nuten kann die mechanische Festigkeit der Übertragungseinrichtung im Bereich der Kopplungseinrichtung deutlich erhöhen. Die Wand kann die Übertragung größerer Kräfte zwischen Übertragungseinrichtung und schwenkbarer Branche ermöglichen.

Bei einem Werkzeug, wie es hier beschrieben ist, sind insbesondere an der schwenkbaren Branche jeweils zwei Kopplungsabschnitte einander gegenüber angeordnet, wobei an der Übertragungseinrichtung jeweils zwei Kopplungsabschnitte einander gegenüber angeordnet sind.

Einander gegenüberliegend angeordnete Kopplungsabschnitte an der Branche sind insbesondere einander zugewandt und an einander zugewandten Oberflächen der Branche angeordnet. Einander gegenüberliegende Kopplungsabschnitte an der Branche sind insbesondere spiegelsymmetrisch zu einer Symmetrieebene angeordnet, wobei die Symmetrieebene gleichzeitig Symmetrieebene der Übertragungseinrichtung sein kann.

Einander gegenüberliegend angeordnete Kopplungsabschnitte an der Übertragungseinrichtung sind insbesondere voneinander abgewandt an voneinander abgewandten Oberflächenbereichen der Übertragungseinrichtung angeordnet. Einander gegenüber angeordnete Kopplungsabschnitte an der Übertragungseinrichtung sind insbesondere spiegelsymmetrisch zu einer Symmetrieebene angeordnet, wobei die Symmetrieebene gleichzeitig Symmetrieebene der schwenkbaren Branche sein kann.

Bei einem Werkzeug, wie es hier beschrieben ist, weist insbesondere die Übertragungseinrichtung nahe ihrem distalen Ende eine Kröpfung auf.

Die Kröpfung ermöglicht eine Anordnung der Kopplungsabschnitte an der Übertragungseinrichtung asymmetrisch zur Längsachse oder zu der Achse, die proximal der Kröpfung Symmetrieachse der Übertragungseinrichtung ist. Insbesondere ist die Längsachse der Übertragungseinrichtung oder die Achse, die proximal der Kröpfung Symmetrieachse der Übertragungseinrichtung ist, zwischen den Kopplungsabschnitten an der Übertragungseinrichtung und der Schwenkachse der schwenkbaren Branche angeordnet. Dies kann eine Verbesserung des Übersetzungsverhältnisses bzw. bei gleicher Kraft auf die Übertragungseinrichtung ein größeres Drehmoment an der schwenkbaren Branche ermöglichen.

Bei einem Werkzeug, wie es hier beschrieben ist, weist das feststehende Bauteil insbesondere einen Kanal auf, wobei die äußere Kontur des Querschnitts der Übertragungseinrichtung in dem für die Anordnung in dem Kanal vorgesehenen Bereich und die innere Kontur des Querschnitts des Kanals derart ausgebildet sind, dass die Übertragungseinrichtung spiel- und reibungsarm in dem Kanal geführt ist, wobei der Kanal durch ein Bauelement gebildet ist, das in einem Hohlraum des feststehenden Bauteils angeordnet ist, und wobei der Querschnitt des Hohlraums größer als der Querschnitt der Übertragungseinrichtung ist.

Das Bauelement ist insbesondere zumindest teilweise hülsen- oder rohrförmig. Die Ausbildung des Kanals in einem Bauelement, das bei der Fertigung des Werkzeugs erst später an das feststehende Bauteil angesetzt werden kann, kann das Einsetzen einer Übertragungseinrichtung mit einer Kröpfung vereinfachen oder überhaupt erst ermöglichen. Insbesondere wird bei der Fertigung des Werkzeugs zunächst das distale Ende der Übertragungseinrichtung von proximal durch den Hohlraum des feststehenden Bauteils geführt und mit der schwenkbaren Branche gekoppelt. Durch den im Vergleich zum Querschnitt der Übertragungseinrichtung größeren Querschnitt des Hohlraums kann die Übertragungseinrichtung in dem Hohlraum geschwenkt bzw. gekippt werden, um die Kopplungsabschnitte am distalen Ende der Übertragungseinrichtung mit den korrespondierenden Kopplungsabschnitten an der schwenkbaren Branche zu koppeln. Erst danach kann das Bauelement, das den Kanal bildet, von proximal an das feststehende Bauteil des Werkzeugs angesetzt werden. Danach führt der Kanal in dem Bauelement die Übertragungseinrichtung derart spielarm, dass die Kopplung zwischen der Übertragungseinrichtung und der schwenkbaren Branche nicht mehr aufgehoben werden kann, solange das den Kanal bildende Bauelement mit dem feststehenden Bauteil verbunden ist.

Ein medizinisches Instrument umfasst ein Werkzeug, wie es hier beschrieben ist, und einen Schaft, der mit dem proximalen Ende des Werkzeugs verbunden oder verbindbar ist.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine schematische Schnittdarstellung eines Werkzeugs für ein medizinisches Instrument;
- Figur 3: eine weitere schematische Schnittdarstellung des Werkzeugs aus Figur 2;
- Figur 4: eine weitere schematische Schnittdarstellung des medizinischen Werkzeugs aus den Figuren 2 und 3;
- Figur 5: eine weitere schematische Schnittdarstellung des Werkzeugs aus den Figuren 2 bis 4;
- Figur 6: eine weitere schematische Schnittdarstellung des Werkzeugs aus den Figuren 2 bis 5;
- Figur 7: eine schematische Schnittdarstellung eines weiteren Werkzeugs;
- Figur 8: eine weitere schematische Schnittdarstellung des Werkzeugs aus Figur 7.

### Beschreibung der Ausführungsformen

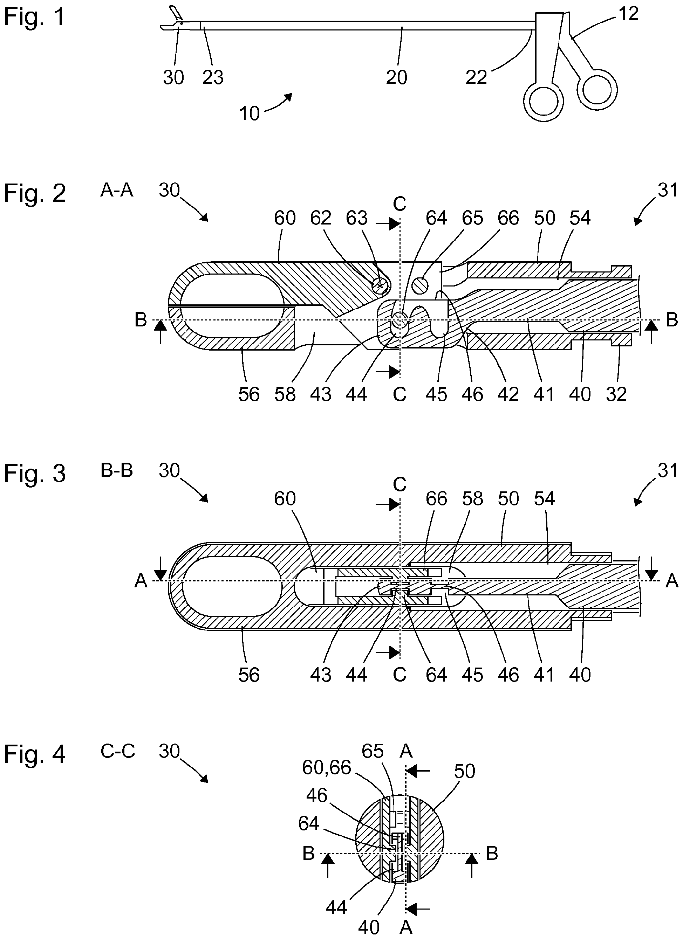

Figur 1 zeigt eine schematische Darstellung eines medizinischen Instruments 10 mit einer Handhabungseinrichtung 12, einem Schaft 20 und einem Werkzeug 30. Das proximale Ende 22 des Schafts 20 ist zerstörungsfrei lösbar oder dauerhaft bzw. nicht zerstörungsfrei lösbar mit der Handhabungseinrichtung 12 mechanisch verbunden. Das distale Ende 23 des Schafts 20 ist zerstörungsfrei lösbar oder dauerhaft bzw. nicht zerstörungsfrei lösbar mit dem Werkzeug 30 mechanisch verbunden.

In dem Schaft 20 ist eine von außen nicht sichtbare und deshalb in Figur 1 nicht dargestellte Übertragungseinrichtung angeordnet. Die Übertragungseinrichtung koppelt einen manuell bewegbaren Teil der Handhabungseinrichtung 12 mit dem Werkzeug 30. Die Übertragungseinrichtung ist zur Übertragung einer Kraft und optional eines Drehmoments zwischen der Handhabungseinrichtung 12 und dem Werkzeug 30 vorgesehen und ausgebildet.

Der Schaft 20 kann gerade oder - abweichend von der Darstellung in Figur 1 - gekrümmt, starr oder flexibel ausgebildet sein.

Figur 2 zeigt eine schematische Darstellung eines Schnitts durch ein Werkzeug 30. Das Werkzeug 30 kann Bestandteil des anhand der Figur 1 dargestellten medizinischen Instruments 10 sein. Alternativ kann das Werkzeug 30 zur dauerhaften bzw. nicht zerstörungsfrei lösbaren oder zur zerstörungsfrei lösbaren Verbindung mit einem distalen Ende eines Schafts zur Bildung eines medizinischen Instruments vorgesehen sein. Die in Figur 2 dargestellte Schnittebene A-A ist parallel zur Längsachse eines mit dem Werkzeug 30 verbundenen oder zu verbindenden Schafts oder - im Falle eines gekrümmten Schafts - zu dessen Längsachse an dessen distalem Ende. In Figur 2 sind die Positionen zweier weiterer Schnittebenen B-B und C-C angedeutet.

Das Werkzeug 30 weist an seinem proximalen Ende 31 eine Kupplung 32 zur lösbaren mechanischen Verbindung mit einem distalen Ende eines Schafts zur Bildung eines medizinischen Instruments auf. Die Kupplung 32 ist beispielsweise für eine Renk- oder Bajonettverbindung ausgebildet.

Das Werkzeug 30 umfasst ferner eine Übertragungseinrichtung 40, deren distaler Endbereich in dem Werkzeug 30 angeordnet und in Figur 2 dargestellt ist. Die Übertragungseinrichtung 40 weist einen Abschnitt 41 mit reduziertem Querschnitt auf, an den sich eine Kröpfung 42 anschließt. Distal der Kröpfung 42 bis zu ihrem distalen Ende 43 ist die Übertragungseinrichtung im Wesentlichen plattenförmig oder quaderförmig. Bei dem dargestellten Beispiel ist die Übertragungseinrichtung 40 auch im Bereich des Abschnitts 41 mit reduziertem Querschnitt im Wesentlichen plattenförmig, und Ecken im Bereich der Kröpfung 42 und am distalen Ende 43 sind abgerundet. Die Platte bzw. der flache Quader, als dessen Ausschnitt der Abschnitt 41, die Kröpfung 42 und der Bereich der Übertragungseinrichtung 40 zwischen der Kröpfung 42 und dem distalen Ende 43 angesehen werden können, erstreckt sich parallel oder im Wesentlichen parallel zur Schnittebene A-A der Figur 2.

Die Übertragungseinrichtung 40 weist nahe ihrem distalen Ende 43 zwei parallele und spiegelsymmetrisch angeordnete erste Nuten 44 auf, von denen bei der Darstellung in Figur 2 nur eine dem Betrachter zugewandt und deshalb sichtbar ist. Ferner weist die Übertragungseinrichtung 40 nahe ihrem distalen Ende 43 und proximal der ersten Nuten 44 zwei parallele und spiegelsymmetrisch zueinander angeordnete zweite Nuten 45 auf, von denen bei der Darstellung in Figur 2 nur eine dem Betrachter zugewandt und deshalb sichtbar ist. Zwischen den beiden parallelen und symmetrisch zueinander angeordneten ersten Nuten 44 und zwischen den beiden parallelen und symmetrisch zueinander angeordneten zweiten Nuten 45 ist eine Wand 46 angeordnet, die sich parallel zur Schnittebene A-A der Figur 2 erstreckt. Auch die ersten Nuten 44 und die zweiten Nuten 45 erstrecken sich jeweils parallel oder im Wesentlichen parallel zur Schnittebene A-A der Figur 2. Die Schnittebene A-A der Figur 2 schneidet eine der beiden ersten Nuten, nämlich die dem Betrachter zugewandte erste Nut 44 und eine der beiden zweiten Nuten, nämlich die dem Betrachter zugewandte zweite Nut 45.

Das Werkzeug 30 umfasst ferner ein feststehendes Bauteil 50 mit einer Durchgangsbohrung. 54. Das feststehende Bauteil 50 bildet bei dem dargestellten Beispiel auch die Kupplung 32 am proximalen Ende 31 des Werkzeugs 30 und eine feststehende Branche 56. Die feststehende Branche 56 ist relativ zu dem proximalen Ende 31 des Werkzeugs 30 und einem mit dem Werkzeug 30 in der vorgesehenen Weise mechanisch verbundenen Schaft nicht bewegbar, insbesondere nicht schwenkbar. Das feststehende Bauteil 50 ist aus wenigen, insbesondere nur zwei oder drei miteinander starr gefügten Bauelementen gebildet.

In der Durchgangsbohrung in dem feststehenden Bauteil 50 ist der in Figur 2 dargestellte distale Endbereich der Übertragungseinrichtung 40 oder ein Teil desselben angeordnet. Die äußere Kontur des Querschnitts der Übertragungseinrichtung 40 proximal des Abschnitts 41 mit reduziertem Querschnitt und die innere Kontur des Querschnitts der Durchgangsbohrung 54 im feststehenden Bauteil 50 sind so ausgebildet, dass die Übertragungseinrichtung 40 in dem feststehenden Bauteil 50 spiel- und reibungsarm geführt ist. Insbesondere ist die Übertragungseinrichtung 40 relativ zu dem feststehenden Bauteil 50 lediglich in Richtung parallel zu ihrer Längsachse und zur Schnittebene A-A innerhalb eines vorbestimmten Bereichs bewegbar. Die Längsachse der Übertragungseinrichtung 40 ist insbesondere die Symmetrieachse, zu der die Übertragungseinrichtung 40 proximal des Abschnitts 41 mit reduziertem Querschnitt rotationssymmetrisch ist, und/oder die Gerade, auf der die Schwerpunkte der Querschnittsflächen der Übertragungseinrichtung 40 in einem Bereich, der sich proximal an dem Abschnitt 41 mit reduziertem Querschnitt anschließt, liegen.

Die Übertragungseinrichtung 40 und die Durchgangsbohrung 54 des feststehenden Bauteils 50 sind insbesondere so ausgebildet (beispielsweise jeweils kreiszylindrisch), dass die Übertragungseinrichtung 40 relativ zu dem feststehenden Bauteil 50 um ihre Längsachse rotierbar wäre, wenn die nachfolgend beschriebene Kopplung mit einem weiteren Bauteil des Werkzeugs 30 dies nicht unterbinden würde. Alternativ können die Übertragungseinrichtung 40 und das feststehende Bauteil 50, insbesondere die Durchgangsbohrung 54, beispielsweise mit nicht rotationssymmetrischen Querschnitten, so ausgebildet sein, dass die Übertragungseinrichtung 40 relativ zu dem feststehenden Bauteil 50 nicht rotierbar ist.

Das Werkzeug 30 umfasst ferner ein schwenkbares Maulteil oder eine schwenkbare Branche 60, die durch ein Gelenk 62 mit dem feststehenden Bauteil 50 schwenkbar verbunden ist. Das Gelenk 62 definiert eine Schwenkachse 63 orthogonal zur Längsachse der Übertragungseinrichtung 40 und orthogonal zur Schnittebene A-A der Figur 2. Das Gelenk 62 wird beispielsweise durch eine Welle gebildet, die sich orthogonal zur Schnittebene A-A der Figur 2 erstreckt.

Die schwenkbare Branche 60 ist relativ zu dem feststehenden Bauteil 50 innerhalb eines vorbestimmten Winkelbereichs um die Schwenkachse 63 schwenkbar. Der vorbestimmte Winkelbereich erstreckt sich zwischen zwei extremen Winkelpositionen der schwenkbaren Branche 60. Eine extreme Winkelposition der schwenkbaren Branche 60 ist in Figur 2 dargestellt. Bei dieser extremen Winkelposition der schwenkbaren Branche 60 liegt diese an der feststehenden Branche 56 des feststehenden Bauteils 50 an.

Die Branche 60 weist zwei einander gegenüberliegende und spiegelsymmetrisch zueinander angeordnete erste Zapfen 64 und zwei einander gegenüberliegende und spiegelsymmetrisch zueinander angeordnete zweite Zapfen 65 an zwei im Wesentlichen parallelen und im Wesentlichen ebenen und spiegelsymmetrisch angeordneten platten- oder wandförmigen Abschnitten 66 auf. Die Schnittebene A-A der Figur 2 ist zwischen den plattenförmigen Abschnitten 66 der schwenkbaren Branche 60 so angeordnet, dass sie einen der beiden ersten Zapfen 64 und einen der beiden zweiten Zapfen 65 schneidet. Der andere erste Zapfen 64 und der andere zweite Zapfen 65 sind bei der Darstellung in Figur 2 verdeckt. Die Zapfen 64, 65 an der schwenkbaren Branche 60 weisen bei dem dargestellten Beispiel jeweils kreisförmige Querschnitte auf.

Die schwenkbare Branche 60, insbesondere die plattenförmigen Bereiche 66 der schwenkbaren Branche 60, und der Bereich der Übertragungseinrichtung 40 zwischen der Kröpfung 42 und ihrem distalen Ende 43 sind in einem Schlitz 58 in dem feststehenden Bauteil 50 angeordnet. Der Schlitz 58 ist bei dem dargestellten Beispiel als schmale und längliche Durchgangsbohrung oder schmale und längliche Ausfräsung, die sich parallel zur Schnittebene A-A der Figur 2 erstreckt, ausgebildet.

Bei der in Figur 2 dargestellten Position der Übertragungseinrichtung 40 und bei der dargestellten Position der schwenkbaren Branche 60 greifen die ersten Zapfen 64 an den plattenförmigen Bereichen 66 der schwenkbaren Branche 60 in die ersten Nuten 44 in der Übertragungseinrichtung 40 ein. Bei anderen Positionen der Übertragungseinrichtung 40 und korrespondierenden anderen Positionen der schwenkbaren Branche 60 greifen alternativ oder zusätzlich die zweiten Zapfen 65 an den plattenförmigen Bereichen 66 der schwenkbaren Branche 60 in die zweiten Nuten 45 an der Übertragungseinrichtung 40 ein.

Die Querschnitte und Positionen der Nuten 44, 45 an der Übertragungseinrichtung 40 und der Zapfen 64, 65 an der schwenkbaren Branche 60 sind so aufeinander abgestimmt, dass die Übertragungseinrichtung 40 und die schwenkbare Branche 60 jederzeit spiel- und reibungsarm gekoppelt sind. Jede lineare Bewegung der Übertragungseinrichtung 40 parallel zu ihrer Längsachse geht somit mit einer Schwenkbewegung der schwenkbaren Branche 60 um ihre Schwenkachse 63 einher.

Bei dem dargestellten Beispiel sind die zweiten Nuten 45 zumindest abschnittsweise gerade, während die ersten Nuten 44 zu den zweiten Nuten 45 hin gekrümmt sind.

Somit bilden die Nuten 44, 45 Kopplungsabschnitte (nämlich im Wesentlichen konkave Kopplungsabschnitte) an der Übertragungseinrichtung 40 und die Zapfen 64, 65 Kopplungsabschnitte (nämlich im Wesentlichen konvexe Kopplungsabschnitte) an der schwenkbaren Branche 60. Die Nuten 44, 45 als Kopplungsabschnitte an der Übertragungseinrichtung 40 und die Zapfen 64, 65 als Kopplungsabschnitte an der schwenkbaren Branche 60 bilden zusammen eine Kopplungseinrichtung zur Kopplung der Übertragungseinrichtung 40 mit der schwenkbaren Branche 60.

Figur 3 zeigt eine schematische Darstellung eines Schnitts entlang der in Figur 2 angedeuteten Ebene B-B durch das Werkzeug 30 aus Figur 2. Die Schnittebene B-B ist parallel zur Längsachse der Übertragungseinrichtung 40 und orthogonal zur Schnittebene A-A der Figur 2. Die Position der Schnittebene A-A der Figur 2 ist in Figur 3 angedeutet.

In dem Schlitz 58, der sich orthogonal zu der Schnittebene B-B der Figur 3 erstreckt, sind die plattenförmigen Bereiche 66 der schwenkbaren Branche 60 und derjenige Bereich der Übertragungseinrichtung 40 nahe deren distalem Ende 43, der die Nuten 44, 45 umfasst, angeordnet. Die Übertragungseinrichtung 40 ist zwischen den plattenförmigen Bereichen 66 der schwenkbaren Branche 60 angeordnet.

Die Schnittebene B-B schneidet die ersten Zapfen 64 an den plattenförmigen Bereichen 66 der schwenkbaren Branche 60. Die ersten Zapfen 64 sind einander gegenüber und spiegelsymmetrisch zueinander angeordnet, so dass sie aufeinander zu ragen. Die ersten Zapfen 64 an der schwenkbaren Branche 60 greifen in die ersten Nuten 44 an der Übertragungseinrichtung 40 ein. Die zweiten Zapfen 65 sind bei der in Figur 3 dargestellten Situation und bei der Betrachtungsrichtung der Figur 3 verdeckt und deshalb nicht dargestellt. Die beiden zweiten Nuten 45 an der Übertragungseinrichtung 40 sind parallel zueinander und spiegelsymmetrisch zueinander angeordnet. Zwischen den beiden ersten Nuten 44 und zwischen den beiden zweiten Nuten 45 ist die Wand 46 vorgesehen, die die Festigkeit der Übertragungseinrichtung im Bereich der Nuten 44, 45 vergrößert, vor allem für Zug- und Druckkräfte parallel zur Längsachse der Übertragungseinrichtung 40.

Figur 4 zeigt eine schematische Darstellung eines Schnitts entlang einer Ebene C-C durch das anhand der Figuren 2 und 3 dargestellte Werkzeug 30. Die Schnittebene C-C ist orthogonal zu der Längsachse der Übertragungseinrichtung 40, orthogonal zu der Schnittebenen A-A der Figur 2 und orthogonal zu der Schnittebene B-B der Figur 3. Die Position der Schnittebene C-C ist in den Figuren 2 und 3 angedeutet.

Die Schnittebene C-C der Figur 4 schneidet beide plattenförmige Bereiche 66 der schwenkbaren Branche 60, die ersten Nuten 44 an der Übertragungseinrichtung 40 und die ersten Zapfen 64 an den plattenförmigen Bereichen 66 der schwenkbaren Branche 60. Die zweiten Zapfen 65 an den plattenförmigen Bereichen 66 der schwenkbaren Branche 60 liegen außerhalb der Schnittebene C-C der Figur 4 und sind im Hintergrund erkennbar. Bei dem dargestellten Beispiel ist die Dicke der Wand 46 zwischen den Nuten 44 kleiner als die Tiefe der Nuten 44.

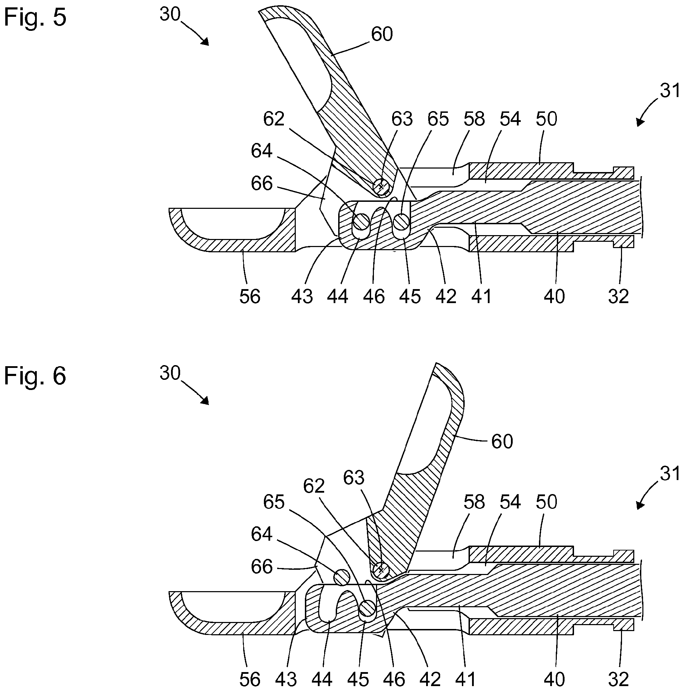

Figur 5 zeigt eine weitere schematische Darstellung eines Schnitts durch das anhand der Figuren 2 bis 4 dargestellte Werkzeug 30. Die Schnittebene der Figur 5 entspricht der Schnittebene A-A der Figur 2.

In Figur 5 ist eine Situation bzw. Konfiguration des Werkzeugs 30 gezeigt, die sich von der in den Figuren 2 bis 4 gezeigten Situation unterscheidet. Insbesondere sind die Übertragungseinrichtung 40 gegenüber der in den Figuren 2 bis 4 gezeigten Situation nach distal verschoben und die schwenkbare Branche 60 durch eine Schwenkbewegung um die Schwenkachse 63 von der feststehenden Branche 56 an dem feststehenden Bauteil 50 wegbewegt. Bei der in Figur 5 gezeigten Situation greifen sowohl die ersten Zapfen 64 an der schwenkbaren Branche 60 in die ersten Nuten 44 an der Übertragungseinrichtung 40 als auch die zweiten Zapfen 65 an der schwenkbaren Branche 60 in die zweiten Nuten 45 an der Übertragungseinrichtung ein.

Figur 6 zeigt eine schematische Darstellung eines Schnitts durch das anhand der Figuren 2 bis 5 dargestellte Werkzeug 30. Die Schnittebene der Figur 6 entspricht der Schnittebene A-A der Figur 2 und der Schnittebene der Figur 5.

Die in Figur 6 dargestellte Situation bzw. Konfiguration unterscheidet sich von den in den Figuren 2 bis 5 gezeigten Situationen dadurch, dass die Übertragungseinrichtung 40 gegenüber der in Figur 5 gezeigten Position noch weiter nach distal verschoben und die schwenkbare Branche 60 gegenüber der in Figur 5 gezeigten Position noch weiter im Uhrzeigersinn um ihre Schwenkachse 63 geschwenkt und damit noch weiter von der feststehenden Branche 56 am feststehenden Bauteil 50 entfernt ist. Bei der in Figur 6 gezeigten Situation greifen nur die zweiten Zapfen 65 an den plattenförmigen Bereichen 66 der schwenkbaren Branche 60 in die zweiten Nuten 45 an der Übertragungseinrichtung 40 ein.

Die in Figur 6 gezeigte Winkelposition der schwenkbaren Branche 60 ist die zu der in Figur 2 gezeigten Position entgegengesetzte extreme Position der schwenkbaren Branche 60.

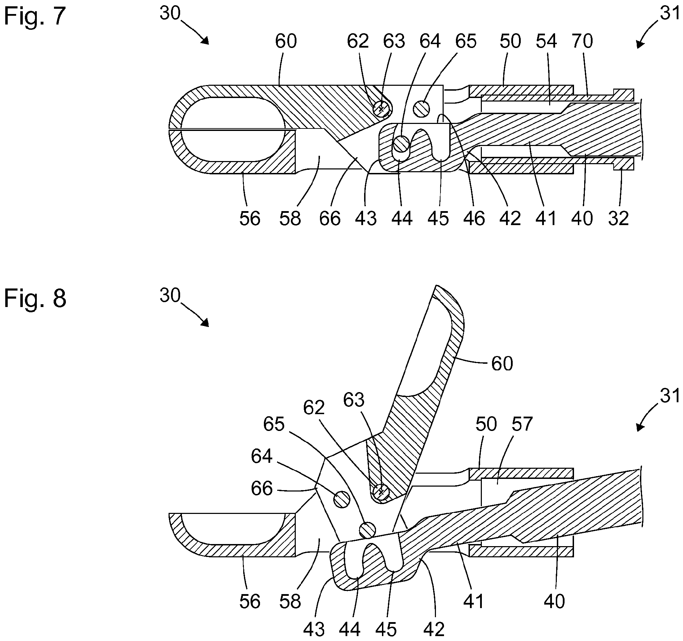

Figur 7 zeigt eine schematische Darstellung eines Schnitts durch ein weiteres Werkzeug 30 eines medizinischen Instruments oder für ein medizinisches Instrument. Die Schnittebene der Figur 7 entspricht der Schnittebene A-A der Figur 2 und den Schnittebenen der Figuren 5 und 6. Das in Figur 7 dargestellte Werkzeug 30 ähnelt in einigen Merkmalen, Eigenschaften und Funktionen dem anhand der Figuren 2 bis 6 dargestellten Werkzeug. Die in Figur 7 gezeigte Situation bzw. Konfiguration entspricht der anhand der Figuren 2 bis 4 dargestellten Situation.

Das in Figur 7 gezeigte Werkzeug 30 unterscheidet sich von dem anhand der Figuren 2 bis 6 dargestellten Werkzeug insbesondere dadurch, dass das feststehende Bauteil 50 aus mehreren Bauelementen gebildet ist. Insbesondere wird das proximale Ende 31 des Werkzeugs 30 mit der Kupplung 32 durch ein Bauelement 70 gebildet. Das Bauelement 70 ist - insbesondere von der Kupplung 32 abgesehen - im Wesentlichen rohr- bzw. hülsenförmig und umschließt die Durchgangsbohrung 54 des feststehenden Bauteils 50. Die innere Oberfläche des Bauelements 70 bildet somit die Oberfläche der Durchgangsbohrung 54, an der die Übertragungseinrichtung 40 anliegt, und die die Übertragungseinrichtung 40 spielund reibungsarm führt. Das Bauelement 70 ist von dem übrigen feststehenden Bauteil 50 separat hergestellt und nach Herstellung der Kopplung zwischen der Übertragungseinrichtung 40 und der schwenkbaren Branche 60 (entsprechend der Beschreibung anhand der Figur 8) an das übrige feststehende Bauteil 50 angesetzt und mit diesem gefügt, beispielsweise durch Laserschweißen.

Figur 8 zeigt eine weitere schematische Darstellung eines Schnitts durch das anhand der Figur 7 dargestellte Werkzeug 30. Die Schnittebene der Figur 8 entspricht der Schnittebene der Figur 7.

In Figur 8 ist eine Situation bzw. Konfiguration während des Zusammensetzens des Werkzeugs 30 dargestellt. Das anhand der Figur 7 beschriebene Bauelement 70 ist noch nicht an das übrige feststehende Bauteil 50 angesetzt. Ein Hohlraum 57 in dem feststehenden Bauteil 50 weist einen Querschnitt auf, der deutlich größer ist als der Querschnitt der Durchgangsbohrung 54 in dem Bauelement 70 (vgl. Figur 7). Dadurch ist die Übertragungseinrichtung 40 bei der in Figur 8 gezeigten Situation noch nicht spielarm in dem feststehenden Bauteil 50 geführt. Deshalb ist die Übertragungseinrichtung 40 relativ zu dem feststehenden Bauteil 50 innerhalb eines vorbestimmten Bereichs, der durch die Querschnitte der Übertragungseinrichtung 40 und des Hohlraums 57 in dem feststehenden Bauteil 50 bestimmt ist, um eine Achse orthogonal zu der Längsachse der Übertragungseinrichtung 40 kippbar.

Das große Spiel der Übertragungseinrichtung 40 in dem Hohlraum 57 in dem feststehenden Bauteil 50 ermöglicht ein Einführen der Übertragungseinrichtung 40 von proximal in den Hohlraum 57 in dem feststehenden Bauteil 50 und ein vollständiges Hindurchführen des distalen Endes 43 und der Kröpfung 42 der Übertragungseinrichtung 40 durch den Hohlraum 57 in dem feststehenden Bauteil 50. Ferner ermöglicht das große Spiel bzw. die Bewegbarkeit der Übertragungseinrichtung 50 in dem Hohlraum 57 in dem feststehenden Bauteil 50 ein Kippen der Übertragungseinrichtung 40 bis zu der in Figur 8 gezeigten Position.

Von der in Figur 8 gezeigten Position der Übertragungseinrichtung 40 aus kann diese zu ihrer in Figur 7 gezeigten vorgesehenen Position bewegt werden, wobei die zweiten Zapfen 65 an den plattenförmigen Bereichen 66 der schwenkbaren Branche 60 in die zweiten Nuten 45 an der Übertragungseinrichtung 40 eingeführt werden. Damit kann eine Situation hergestellt werden, die der anhand der Figur 6 oder auch der anhand der Figur 5 gezeigten entspricht, und bei der die Übertragungseinrichtung 40 und die schwenkbare Branche 60 durch Eingriff der ersten Zapfen 64 in die ersten Nuten 44 und/oder der zweiten Zapfen 65 in die zweiten Nuten 45 formschlüssig gekoppelt sind.

Danach kann das Bauelement 70 des feststehenden Bauteils 50 (vgl. Figur 7) von proximal in den Hohlraum 57 eingeführt und mit dem übrigen feststehenden Bauteil 50 gefügt werden, um die in Figur 7 gezeigte Situation herzustellen.

Die anhand der Figuren 2 bis 8 dargestellten Werkzeuge können vielfach variiert werden. Beispielsweise können dritte Zapfen zum Eingriff in korrespondierende dritte Nuten, vierte Zapfen zum Eingriff in korrespondierende vierte Nuten etc. vorgesehen sein.

Ferner können die Übertragungseinrichtung 40 und die schwenkbare Branche 60 nicht spiegelsymmetrisch ausgebildet sein. Insbesondere können Nuten 44, 45 und Zapfen 64, 65 an gegenüberliegenden Seiten an verschiedenen Stellen und zur Kopplung in verschiedenen Winkelbereichen vorgesehen sein. Ferner können nur an einer Seite der Übertragungseinrichtung 40 Nuten 44, 45 und nur an einem plattenförmigen Bereich 66 der schwenkbaren Branche 60 Zapfen 64, 65 vorgesehen sein. Dabei kann der zweite plattenförmige Bereich 66 entfallen.

Ferner kann an der schwenkbaren Branche 60 lediglich ein plattenförmiger Bereich 66 vorgesehen sein. An diesem plattenförmigen Bereich 66 können lediglich an einer Seite Zapfen 64, 65 oder an beiden voneinander abgewandten Seiten Zapfen 64, 65 vorgesehen sein. Dabei kann der distale Endbereich der Übertragungseinrichtung 40 gabelförmig den plattenförmigen Bereich 66 der schwenkbaren Branche 60 umgreifen. In diesem Fall sind an einem oder an beiden Schenkeln des distalen Endbereichs der Übertragungseinrichtung 40 an den einander zugewandten Oberflächenbereichen Nuten 44, 45 vorgesehen.

Ferner können alternativ oder zusätzlich zu einer oder mehreren Nuten 44, 45 an der Übertragungseinrichtung 40 und korrespondierenden Zapfen 64, 65 an der schwenkbaren Branche 60 eine oder mehrere Nuten in der schwenkbaren Branche 60 und eine entsprechende Anzahl korrespondierender Zapfen an der Übertragungseinrichtung 40 vorgesehen sein.

Ferner können zwei oder mehr schwenkbare Branchen 60 vorgesehen sein, die jeweils durch Formschluss von Kopplungsabschnitten an der Übertragungseinrichtung 40 und Kopplungsabschnitten an jeder einzelnen schwenkbaren Branche 60 mit der Übertragungseinrichtung 40 gekoppelt sein können.

### Bezugszeichen

- 10: Medizinisches Instrument
- 12: Handhabungseinrichtung des medizinischen Instruments 10
- 20: Schaft des medizinischen Instruments 10
- 22: proximales Ende des Schafts 20
- 23: distales Ende des Schafts 20
- 30: Werkzeug am distalen Ende 23 des Schafts 20
- 31: proximales Ende des Werkzeugs 30
- 32: Kupplung am proximalen Ende 31 des Werkzeugs 30
- 40: Übertragungseinrichtung zum Übertragen einer Kraft zu der schwenkbaren Branche 60
- 41: Abschnitt der Übertragungseinrichtung 40 mit reduziertem Querschnitt
- 42: Kröpfung der Übertragungseinrichtung 60
- 43: distales Ende der Übertragungseinrichtung 40
- 44: erste Nut als erster, konkaver Kopplungsabschnitt nahe dem distalen Ende 43 der Übertragungseinrichtung 40
- 45: zweite Nut als zweiter, konkaver Kopplungsabschnitt nahe dem distalen Ende 43 der Übertragungseinrichtung 40
- 46: Wand zwischen ersten Nuten 44 und zwischen zweiten Nuten 45
- 47: durch die erste Nut 44 definierter Pfad des ersten Zapfens 64 in der ersten Nut 44
- 50: feststehendes Bauteil des Werkzeugs 30
- 54: Durchgangsbohrung in dem feststehenden Bauteil 50, durch die die Übertragungseinrichtung 40 hindurch ragt
- 56: feststehende Branche am feststehenden Bauteil 50
- 57: Hohlraum in dem feststehenden Bauteil 50 zur Aufnahme des Bauelements 70
- 58: Schlitz zur Aufnahme der plattenförmigen Bereiche 66 der schwenkbaren Branche 60
- 60: schwenkbare Branche des Werkzeugs 30
- 62: Gelenk zwischen dem feststehenden Bauteil 50 und der schwenkbaren Branche 60
- 63: Schwenkachse der schwenkbaren Branche 60, durch das Gelenk 62 definiert
- 64: erster Zapfen als erster, konvexer Kopplungsabschnitt an der schwenkbaren Branche 60
- 65: zweiter Zapfen als zweiter, konvexer Kopplungsabschnitt an der schwenkbaren Branche 60
- 66: plattenförmiger Bereich der schwenkbaren Branche 60
- 70: Bauelement des feststehenden Bauteils 50 mit der Durchgangsbohrung 54 und der Kupplung 32

## Patentansprüche

1. **Werkzeug** (30) für ein medizinisches Instrument (10), mit:
einem **feststehenden Bauteil** (50);
einer **Branche** (60), die relativ zu dem feststehenden Bauteil (50) um eine Schwenkachse (63) schwenkbar ist;
einer **Übertragungseinrichtung** (40) zum Übertragen einer Kraft zu der schwenkbaren Branche (60);
einer **Kopplungseinrichtung** (44, 45, 64, 65) zum Koppeln der Übertragungseinrichtung (40) mit der schwenkbaren Branche (60) derart, dass eine Translation der Übertragungseinrichtung (40) mit einer Schwenkbewegung der schwenkbaren Branche (60) um ihre Schwenkachse (63) einhergeht,
wobei die Kopplungseinrichtung mehrere **Kopplungsabschnitte** (64, 65) an der schwenkbaren Branche (60) und mehrere Kopplungsabschnitte (44, 45) an der Übertragungseinrichtung (40) umfasst,
wobei jeder Kopplungsabschnitt (64, 65) an der schwenkbaren Branche (60) einem korrespondierenden Kopplungsabschnitt (44, 45) an der Übertragungseinrichtung (40) zugeordnet ist,
wobei die Kopplungsabschnitte (44, 45, 64, 65) so angeordnet und ausgebildet sind, dass die Kopplung von schwenkbarer Branche (60) und Übertragungseinrichtung (40) abhängig von den Positionen von schwenkbarer Branche (60) und Übertragungseinrichtung (40) durch unterschiedliche Paare korrespondierender Kopplungsabschnitte (44, 64, 45, 65) erfolgt,
**dadurch gekennzeichnet, dass**
ein Kopplungsabschnitt (64, 65) an der schwenkbaren Branche (60) einen **Zapfen** oder einen Stift oder einen stabförmigen Abschnitt umfasst, und wobei die Schwenkachse orthogonal zu einer vorgesehenen Bewegungsrichtung der Übertragungseinrichtung (40) ist und
der Querschnitt des Zapfens, Stifts oder stabförmigen Abschnitts kreisförmig ist.

2. Werkzeug (30) nach dem vorangehenden Anspruch, bei dem ein Kopplungsabschnitt (44, 45) an der Übertragungseinrichtung (40) eine Nut oder einen Schlitz in der Übertragungseinrichtung (40) umfasst.

3. Werkzeug (30) nach einem der vorangehenden Ansprüche, bei dem jeweils zwei als **Zapfen** ausgebildete Kopplungsabschnitte (64, 65) an der schwenkbaren Branche (60) einander **gegenüber** angeordnet sind,
jeweils zwei als **Nuten** ausgebildete Kopplungsabschnitte (44, 45) an der Übertragungseinrichtung (40) einander **gegenüber** angeordnet sind.

4. Werkzeug (30) nach dem vorangehenden Anspruch, bei dem die Kopplungsabschnitte paarweise spiegelsymmetrisch zu einer Symmetrieebene angeordnet sind.

5. Werkzeug (30) nach einem der Ansprüche 3 und 4, bei dem eine **Wand** (46) zwischen einander gegenüberliegenden Nuten (44, 45) an der Übertragungseinrichtung (40) angeordnet ist.

6. Werkzeug (30) nach einem der vorangehenden Ansprüche, bei dem
an der schwenkbaren Branche (60) jeweils zwei Kopplungsabschnitte (64, 65) einander **gegenüber** angeordnet sind,
an der Übertragungseinrichtung (40) jeweils zwei Kopplungsabschnitte (44, 45) einander **gegenüber** angeordnet sind.

7. Werkzeug (30) nach einem der vorangehenden Ansprüche, bei dem die Übertragungseinrichtung (40) nahe ihrem distalen Ende eine **Kröpfung** (42) aufweist.

8. Werkzeug (30) nach einem der vorangehenden Ansprüche, bei dem
das feststehende Bauteil (50) einen Kanal (54) aufweist,
die äußere Kontur des Querschnitts der Übertragungseinrichtung (40) in dem für die Anordnung in dem Kanal (54) vorgesehenen Bereich und die innere Kontur des Querschnitts des Kanals (54) derart ausgebildet sind, dass die Übertragungseinrichtung (40) spiel- und reibungsarm in dem Kanal (54) geführt ist,
der Kanal durch ein Bauelement (70) gebildet ist, das in einem Hohlraum (57) des feststehenden Bauteils (50) angeordnet ist,
der Querschnitt des Hohlraums (57) größer als der Querschnitt der Übertragungseinrichtung (40) ist.

9. **Medizinisches Instrument** (10) mit
einem Werkzeug (30) nach einem der vorangehenden Ansprüche;
einem Schaft (20), der mit dem proximalen Ende (31) des Werkzeugs verbunden oder verbindbar ist.

## Claims

1. **Tool** (30) for a medical instrument (10), with:
a **stationary component** (50);
a **branch** (60) which is pivotable relative to the stationary component (50) about a pivot axis (63);
a **transmission device** (40) for transmitting a force to the pivotable branch (60);
a **coupling device** (44, 45, 64, 65) for coupling the transmission device (40) to the pivotable branch (60) in such a way that a translation of the transmission device (40) accompanies a pivoting movement of the pivotable branch (60) about its pivot axis (63),
the coupling device comprising several **coupling portions** (64, 65) on the pivotable branch (60) and several coupling portions (44, 45) on the transmission device (40),
each coupling portion (64, 65) on the pivotable branch (60) being assigned to a corresponding coupling portion (44, 45) on the transmission device (40),
the coupling portions (44, 45, 64, 65) being arranged and designed such that the coupling of the pivotable branch (60) and the transmission device (40) is carried out, depending on the positions of the pivotable branch (60) and the transmission device (40), by different pairs of corresponding coupling portions (44, 64, 45, 65),
**characterized in that** a coupling portion (64, 65) on the pivotable branch (60) comprises a **journal** or a pin or a rod-shaped portion, and the pivot axis being orthogonal with respect to a provided movement direction of the transmission device (40), and
the cross section of the journal, pin or rod-shaped portion being circular.

2. Tool (30) according to the preceding claim, in the case of which a coupling portion (44, 45) on the transmission device (40) comprises a **groove** or a slit in the transmission device (40).

3. Tool (30) according to either of the preceding claims, in the case of which in each case two coupling portions (64, 65) which are configured as **journals** are arranged **opposite** one another on the pivotable branch (60), and in each case two coupling portions (44, 45) which are configured as **grooves** are arranged **opposite** one another on the transmission device (40).

4. Tool (30) according to the preceding claim, in the case of which the coupling portions are arranged in pairs mirror-symmetrically with respect to a plane of symmetry.

5. Tool (30) according to either of Claims 3 and 4, in the case of which a **wall** (46) is arranged between mutually opposite grooves (44, 45) on the transmission device (40) .

6. Tool (30) according to one of the preceding claims, in the case of which
two coupling portions (64, 65) are in each case arranged **opposite** one another on the pivotable branch (60), and
two coupling portions (44, 45) are in each case arranged **opposite** one another on the transmission device (40).

7. Tool (30) according to one of the preceding claims, in the case of which the transmission device (40) has an **offset** (42) near its distal end.

8. Tool (30) according to one of the preceding claims, in the case of which
the stationary component (50) has a channel (54),
the outer contour of the cross section of the transmission device (40), in the area provided for arranging in the channel (54), and the inner contour of the cross section of the channel (54) are configured in such a way that the transmission device (40) is guided in the channel (54) with little play and little friction,
the channel is formed by way of a structural element (70) which is arranged in a cavity (57) of the stationary component (50), and
the cross section of the cavity (57) is greater than the cross section of the transmission device (40).

9. **Medical instrument** (10) with
a tool (30) according to one of the preceding claims;
a shank (20), which is connected or can be connected to the proximal end (31) of the tool.

## Revendications

1. Outil (30) pour un instrument médical (10), comportant :
un composant fixe (50) ;
une branche (60) qui est pivotante autour d'un axe de pivotement (63) par rapport au composant fixe (50) ;
un dispositif de transmission (40) servant à la transmission d'une force à la branche pivotante (60) ;
un dispositif d'accouplement (44, 45, 64, 65) servant à l'accouplement du dispositif de transmission (40) à la branche pivotante (60) de telle sorte qu'une translation du dispositif de transmission (40) soit accompagnée d'un mouvement de pivotement de la branche pivotante (60) autour de son axe de pivotement (63),
le dispositif d'accouplement comportant plusieurs parties d'accouplement (64, 65) sur la branche pivotante (60) et plusieurs parties d'accouplement (44, 45) sur le dispositif de transmission (40),
chaque partie d'accouplement (64, 65) sur la branche pivotante (60) étant associée à une partie d'accouplement (44, 45) correspondante sur le dispositif de transmission (40),
les parties d'accouplement (44, 45, 64, 65) étant agencées et réalisées de telle sorte que l'accouplement de la branche pivotante (60) et du dispositif de transmission (40) s'effectue, en fonction des positions de la branche pivotante (60) et du dispositif de transmission (40), au moyen de différentes paires de parties d'accouplement (44, 64, 45, 65) correspondantes,
**caractérisé en ce**
**qu'**une partie d'accouplement (64, 65) sur la branche pivotante (60) comporte un tenon ou une goupille ou une partie en forme de tige, et
l'axe de pivotement étant perpendiculaire à une direction de déplacement prévue du dispositif de transmission (40) et
la section transversale du tenon, de la goupille ou de la partie en forme de tige étant circulaire.

2. Outil (30) selon l'une des revendications précédentes, dans lequel une partie d'accouplement (44, 45) sur le dispositif de transmission (40) comporte une rainure ou une fente dans le dispositif de transmission (40) .

3. Outil (30) selon l'une des revendications précédentes, dans lequel
respectivement deux parties d'accouplement (64, 65) réalisées sous forme de tenons sur la branche pivotante (60) sont agencées en regard l'une de l'autre,
respectivement deux parties d'accouplement (44, 45) réalisées sous forme de rainures sur le dispositif de transmission (40) sont agencées en regard l'une de l'autre.

4. Outil (30) selon l'une des revendications précédentes, dans lequel les parties d'accouplement sont agencées par paires en symétrie miroir par rapport à un plan de symétrie.

5. Outil (30) selon l'une des revendications 3 et 4, dans lequel une paroi (46) est agencée entre des rainures (44, 45) en regard l'une de l'autre sur le dispositif de transmission (40).

6. Outil (30) selon l'une des revendications précédentes, dans lequel
deux parties d'accouplement (64, 65) sont agencées respectivement en regard l'une de l'autre sur la branche pivotante (60),
deux parties d'accouplement (44, 45) sont agencées respectivement en regard l'une de l'autre sur le dispositif de transmission (40).

7. Outil (30) selon l'une des revendications précédentes, dans lequel le dispositif de transmission (40) comprend un coude (42) près de son extrémité distale.

8. Outil (30) selon l'une des revendications précédentes, dans lequel
le composant fixe (50) comprend un canal (54),
le contour extérieur de la section transversale du dispositif de transmission (40) dans la région prévue pour l'agencement dans le canal (54) et le contour intérieur de la section transversale du canal (54) sont réalisés de telle sorte que le dispositif de transmission (40) est guidé dans le canal (54) avec faible jeu et faible friction,
le canal est formé par un élément structural (70) qui est agencé dans une cavité (57) du composant fixe (50),
la section transversale de la cavité (57) est plus grande que la section transversale du dispositif de transmission (40) .

9. Instrument médical (10) comportant un outil (30) selon l'une des revendications précédentes ; une tige (20) qui est reliée ou peut être reliée à l'extrémité proximale (31) de l'outil.
